Europäisches Patentamt

European Patent Office

Office européen des brevets

(11) Publication number: **0 420 979 B1**

## EUROPEAN PATENT SPECIFICATION

(45) Date of publication of patent specification: **19.01.94**  (51) Int. Cl.5: **A61K 37/18**

(21) Application number: **89902067.1**

(22) Date of filing: **02.02.89**

(86) International application number:
**PCT/JP89/00107**

(87) International publication number:
**WO 89/06970 (10.08.89 89/18)**

(54) LIPID METABOLISM IMPROVING AGENT AND METHOD OF ITS USE.

(30) Priority: **02.02.88 JP 23624/88**

(43) Date of publication of application:
**10.04.91 Bulletin 91/15**

(45) Publication of the grant of the patent:
**19.01.94 Bulletin 94/03**

(84) Designated Contracting States:
**AT BE CH DE FR GB IT LI NL SE**

(56) References cited:
EP-A- 0 044 032
JP-A- 5 976 022
JP-A-58 225 026
JP-A-60 164 496

(73) Proprietor: **HANKYU-KYOEI BUSSAN CO., LTD.**
**Hankyu Building 7-go, 8-ban**
**Kakuta-cho, Kita-ku Osaka-shi, Osaka**
**531(JP)**

(72) Inventor: **KAGAWA, Kyoichi**
**608-go, 9-ban, Toyohara-cho**
**Ibaraki-shi Osaka 567(JP)**

Inventor: **KANEDA, Takaya**
**19-go, 21-ban, 5-chome**
**Takeoka**
**Kagoshima-shi Kagoshima 890(JP)**
Inventor: **TADOKORO, Tetsuo**
**107, 19-go, 5-ban**
**Aoki-cho**
**Nishinomiya-shi Hyoko-Ken 662(JP)**
Inventor: **MATSUMURA, Yoshikazu, Ueda**
**Chemical Ind.Co.Ltd.**
**6-6-go, 2-ban, 1-chome,**
**Takayanagi**
**Neyagawa-shi, Osaka 572(JP)**

(74) Representative: **Brommer, Hans Joachim,**
**Dr.-Ing. et al**
**Patentanwälte Dipl.-Ing. R. Lemcke**
**Dr.-Ing. H.J. Brommer,**
**Postfach 40 26**
**D-76025 Karlsruhe (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a lipid metabolism promoting agent and its usage useful for preventing or treating high blood pressure or arteriosclerosis in the human body or for improving meat quality of cattles or fish in the field of the livestock industry and the marine products industry.

For the prevention or treatment of high blood pressure and arteriosclerosis, it is known, in combination with restriction of fat intake, to administrate medicines such as dextran sulphate for suppressing the digestion and absorption of lipid or for promoting the blood lipoprotein lipase activity. As a lipid absorption suppressing agent, such medicine as Nicomole is known. As a lipid metabolism promoting agent or pancreatic elastase as well as the above-noted dextran sulphate is known.

However, such medicines as Nicomole have the inevitable disadvantages of undesirable side effects. On the other hand, in the fields of livestock and marine products industries, it has been a common practice to give to the cattles or fish such high-calory type feeds as contain a large amount of fat and protein, whereby the grown animals tend to have excessive amount of fat in their meat. In recent years; however, the health-conscious populations's preference is for lean meat, wanting to appropriately control the amount and quality of fat intake. That is to say, what is desired today is to restrict fat intake and excess fat accumulation in the body and further to improve the quality of the accumulated body fat, without inviting unfavorable side effects.

The primary object of the present invention is to overcome the above-described drawbacks of the prior art and to provide a lipid metabolism promoting agent and its usage which may advantageously suppress excess fat intake and excess fat accumulation in the body and further to improve the quality of the accumulated body fat without inviting unfavorable side effects.

For accomplishing the above object, a use of a mixture related to the present invention is provided by low molecular peptide prepared through hydrolysis of a protein or a protein-containing material by a protease or an acid, the mixture having an average amino acid chain length of approximately 3 to 4.

On the other hand, a usage of lipid metabolism promoting agent also related to the present invention comprises the step of administrating, by 0.1 to 50 wt.% relative to a total fat intake amount, the lipid metabolism promoting agent including as an effective component thereof low molecular peptide prepared through hydrolysis of a protein or a protein-containing material, with the peptide having approximately 3 to 4 average amino acid chain length. Accordingly, with ordinary food stuff, the administration amount of the agent may be as little as about 1 wt. % relative to the total food stuff intake. Further, even with fatty food stuff having lipid content of approximately 20 to 40 %, the administration may be no greater than 4 to 20. wt. % relative to the total food intake.

Through intensive research and varied experiments, the inventors of the present invention have found out that the 0.1 to 50 wt.% or more preferrably 1 to 30 wt.% addition relative to the total fat intake of the low molecular peptide having the average amino acid chain length of 3 to 4 is most effective for suppressing the in vitro lipase activity to the lipid. In addition, it has become clear also that the above agent when fed alone or in combination with food stuff or feed reduces fat digestion and absorption, decreases triglyceride concentration and cholesterol concentration in the blood and further reduces fat content and fat droplets in the liver without affecting amount of protein contained in the blood or the liver tissue. Moreover, the agent has proved to reduce the amount of excess body fat and to improve the composition quality of lipids.

In contrast to this it is known in the art (EP-A-0044032) to use a nutrient agent comprising low-molecular weight peptide mainly based on dipeptides and tripeptides in medical treatment for - among other things - remarkably increasing the gain of body weight, which is extremely different from the goal of the present invention.

It has been a common knowledge among those skilled in the art that an intake of high protein may bring about a reduction in excess body fat. However, in order to achieve this effect, the high-protein-intake must be no less than 40 % in comparison with about 20 % intake in the case of normal diet. In sharp contrast to this, the agent of the present invention is needed by only 1 wt. % for achieving its distinguished effects of fat digestion and absorption suppression and of body fat reduction. Also, the usage of the lipid metabolism promoting agent of the present invention clearly distinguishes from that of commercially available low molecular peptides which are invariably used as a protein source which is effectively absorbed. In other words, the agent of the invention differs from the conventional low molecular peptide agents both in dosage and in object.

In manufacturing the low molecular peptide with the average amino acid chain length of 3 to 4 with protease, the ratio between the protease and the protein is 0.1 to 5 units of pretease relative to 1 mg of protein. The manufacturing operation is carried out for 3 to 48 hours, preferrably 16 to 30 hours, at the temperature of 20 to 70 degrees Celsius, preferrably 40 to 60 degrees Celsius. On unit of protease activity is defined as the amount of enzyme that produces non-proteinaous substances equivalent to 1 µg of tyrosine colored with Folin's reagent, in 1 minute at 30 degrees Celsius and in the optimum pH of the enzyme using milk caseine as the substrate.

One example manufacturing method of the low molecular peptide will be described next. First, a solid matter of an appropriate protein or protein-containing substance, the protein being of an animal, plant or a micro-organism origin, is suspended in water at 5 to 30 w/v %, and the pH is adjusted to the optimum pH of the protease used by an acid or an alkaline. Second, protease is added to the mixture at one time or in succession, and allows the reaction mixture to stand for 3 to 48 hours at the temperature between 20 to 70 degrees Celsius.

Thus obtained low molecular peptide solution is dried by a spray-drier, or alternatively, the low molecular peptide solution is added with an appropriate amount of an extending agent such as carboxymethyl cellulose or dextrin and then dried, whereby a lipid metabolism promoting agent may be obtained.

The above-described lipid metabolism promoting agent of the present invention, when taken by 0.1 to 50 % or peferrably 1 to 30 % relative to a total fat intake weight, is capable of effectively suppressing digestion and absorption of fat and of improving lipid metabolism without incurring unfavorable side effects. As the result, the agent will prove useful for prevention and treatment of high blood pressure, arteriosclerosis or obesity in the human body. In additon, the agent, if added to the feeds, may be also effectively utilized in the fields of livestock or marine products industry for the purpose of limiting excessive fat accumulation in the animal meat and for improving the composition quality of the body fat of the animal.

The low molecular peptide contained as an effective component in the lipid metabolism promoting agent related to the present invention is rendered the low molecular type of about 3 to 4 average amino acid chain length by hydrolyzing a protein or a protein-containing material with a protease or an acid. This low molecular peptide can additionally contain amino acid, dipeptide or a high molecular peptide. Alternately, the low molecular peptide may be refined so as to eliminate all or most of such amino acid or high molecular peptide. Preferrably, in the low molecular peptide, the fraction of the 3 to 4 average amino acid chain length should be contained by no less than 50 % therein. This low molecular peptide remains without loss of its inhibiting action for lipase, even if it is treated with an autoclave (121 degrees Celsius, 1.1 $kg/cm^2$ , 30 min.), or is heated for 30 minutes in a boiling water bath.

The above-noted protease is originated from such micro-organisms as <u>Aspergillus</u> <u>niger</u>, <u>Batillus</u> <u>subtilis</u>. The protease may be any single one of or a combination of an acid protease, neutral protease or an alkaline protease and may be also either of or a combination of an animal-origin type or a plant-origin type. As the acid, an inorganic acid such as hydrocholoric acid or sulfuric acid is employed. It is to be noted; however, that a protease rather than such acid is preferred for efficiently producing the low molecular peptide of about 3 to 4 average amino acid chain length.

Next, some specific manufacturing methods of the low molecular peptide will be particularly described.

Where, an average amino acid chain length (L) of the product is expressed by the following equation:

$$L = \frac{\partial\text{-amino group in the complete hydrolysate of the product with HCl}}{\partial\text{-amino group in the product}}$$

The amount of $\alpha$-amino group liberated was determined by the ninhydrin method. The complete hydrolysis was carried out in 6N hydrocholoric acid at 110 degrees Celsius for 24 hours. Also, distribution of molecular weight of the product was examined by a gel filtration using Toyopearl HW-40S (column: 2.2 cm x 50 cm, solvent: 0.1 M acetate buffer solution pH 5.7).

a) 250 liter of water was added to 100 kg of red blood corpuscles of cattle. The pH of the mixture was adjusted to 2.8 with phosphoric acid. Then, to this mixture, $2 \times 10^7$ units of acid protease originated from <u>Aspergillus niger</u> were added and incubated at 50 degrees Celsius for 20 hours.

After this reaction, the reaction mixture was heated at 80 degrees Celsius for 30 minutes so as to stop the reaction. Then, the mixture was added with calcium hydroxide suspended with water so as to adjust the pH to 6.5 and further added with 10 kg of diatomaceous earth. The mixture was then filtered by a filter press, and the filtered liquid was spray-dried to produce 23 kg of powder. The average amino acid chain length of this powder was measured to be 3.6. Also, a further measurement on molecular weight distribution revealed that the fraction with 3 to 4 average amino acid chain length occupied approximately 65 wt. % in this product.

b) 50 kg of defatted fish powder was suspended in 200 liter of water. Then, the pH of the suspension liquid was adjusted to 2. 8 with phosphoric acid. And, to this mixture, $3 \times 10^7$ units of acid protease described in the above a), was added, and 21 kg of powder were obtained. The average amino acid chain length of this powder was measured to be 3.5. Also, a further measurement on molecular weight distribution revealed that the fraction with 3 to 4 average amino acid chain length occupied approximately 63 wt. % in the product.

c) 40 kg of soya protein isolated was suspended in 200 liter of water. Then, the pH of the suspension mixture was adjusted to 2. 8 with phosphoric acid. And, to this mixture, $3 \times 10^7$ units of acid protease from Asperigillus niger were added. Through the same process described in the above a), 27 kg of powder was obtained. The average amino acid chain length of this powder was measured to be 3.6. Also, a further measurement on molecular weight distribution revealed that the fraction with 3 to 4 average amino acid chain length occupied approximately 67 wt. % in the product.

From the above experimental results, it may be seen that the low molecular peptide with the average amino acid chain lengh of approximately 3 to 4 may be readily manufactured.

Thus prepared low molecular peptides were compared with each other in their acquired lipid metabolism promoting effects. The comparisons revealed that the low molecular peptide based on the soya protein isolated achieves a weaker effect than the others based on the animal proteins and further that the efficacy of the peptide differs depending on the kind of employed protease to hydrolyze a certain protein.

Then, the effects of the low molecular peptide contained as the effective component in the lipid metabolism promoting agent of the invention were confirmed with animal experiments. First, a suppression of the fat digestion and absorption was seen from histogicals observation as a decrease in the lipid droplets existent in the intestinal epitherial cells after oral administration of olive oil. It was also observed that the blood lipid level, especially the neutral lipid level, was suppressed. Further, through an animal testing by four weeks of free feeding of the agent, it was observed that the peptide reduced the body fat weight and also improved its lipid composition. That is to say, the administration of the low molecular peptide has proved to be effective for suppressing lipid digestion and absorption and for improving the lipid composition quality.

Then, a closer observation was made on the above effect of the low molecular peptide as distinguished between its suppressing effect to lipid digestion by a single administration and its reducing effect to the body fat after repeated administration over a long term. The observation revealed that some of the various low molecular peptides are the more effective by a single administration while others are the more effective after the long term use. From this fact, it may be reasonably assumed that a desired effect may be intensified through proper selection among the kinds of peptides and dosage of each of the peptide.

Next, there will be detailed experiments and applications conducted by the present inventor to confirm the effect of the lipid metabolism promoting agent of the invention.

(Experiment 1)

In order to investigate the effect of the low molecular peptide acid to in vitro lipase activity, 100 ng/ml to 1mg/ml of low molecular peptide with the average amino acid chain length of 3 to 4 approximately was added to about 0.3 unit/ml of pancreatic lipase and 0.125 ml/ml of substrate olive oil. It is to be noted here that one unit of lipase activity is defined as the amount of an enzyme that produces 1 $\mu$mol of fatty acid in 1 minute at 37 degrees Celsius.

The experiments were conducted respectively for the low molecular peptide obtained from soya protein isolated, the low molecular peptide obtained from fish powder, low molecular peptide obtained from red blood corpuscle, and the peptide having 15 average chain length obtained from red blood corpuscle by the manufacturing method (described in the foregoing manufacturing method a) except that the reaction time period was reduced this time to 1 hour). The results, as shown in Table I showing the activity ratio (%), show that the lipase activity was inhibited by the addition of the low molecular peptides.

(Experiment 2)

In order to investigate the effect of the low molecular peptide to fat digestion and absorption during an intake of fat, 5 mg of the red blood corpuscle based low molecular peptide, which was proved to provide the weakest inhibitory action for lipase, along with 250 mg of olive oil was given to five-week oil ICR strain

4

EP 0 420 979 B1

male mice (weighing about 20 g) by gastric tube. Then, after 120 minutes, cholesterol and triglyceride in the blood plasma were measured. The results, as shown in Table II, indicate that in spite of the feeding of olive oil the low molecular peptide rendered the both cholesterol and triglyceride values lower than those in the reference animals given olive oil alone.

(Experiment 3)

In order to investigate the effect of repeated administration of the low molecular peptide to the fat digestion and absorption, 1 wt. % of the low molecular peptide used in the above Experiment 2 was mixed into a powderly feed (raw fat content: 5%, manufactured by Oriental Yeast Inc. Ltd.,) and the mixutre was freely given to 7-week oid ICR strain male mice (weighing about 30 g) by gastric tube for 14 days. Then, on the 15th day, 250 mg of olive oil was forcibly given to the animals by a gastric tube. After a lapse of 90 minutes, cholesterol and triglyceride in the blood plasma were measured. The results, as shown in Table III, indicate that both of the values after the 14 days of repeated administrations of the low molecular peptide were lower than those in reference mice and further that any rises in these values due to the feeding of olive oil were completely suppressed by the simultaneous administration of the low molecular peptide.

Table I

| (Experiment 1) (% of activity) | | | | |
|---|---|---|---|---|
| peptide concentration log (g/ml) | low molecular peptide | | | peptide |
| | soy | fish | red cell | red cell |
| -7 | 36 | 52 | 41 | 66 |
| -6 | 0 | 0 | 19 | 55 |
| -5 | -- | -- | 0 | 50 |
| -4 | 25 | 42 | 53 | -- |
| -3 | 32 | 47 | 92 | -- |

Table II

| (Experiment 2) | | |
|---|---|---|
| treatment | cholesterol (mg/dl) | triglyceride (mg/dl) |
| no treatment | 114 | 99 |
| olive oil alone | 124 | 692 |
| both olive oil and red blood corpscle peptide | 93 | 77 |

Table III

| (Experiment III) | | |
|---|---|---|
| treatment | cholesterol (mg/dl) | triglyceride (mg/dl) |
| no treatment | 100 | 99 |
| 14 days administration of red blood corpuscle peptide | 90 | 60 |
| olive oil alone | 132 | 328 |
| both olive oil and 5 mg of red blood corpuscle peptide | 110 | 140 |
| both olive oil and 50 mg of red blood corpuscle peptide | 90 | 100 |

(Experiment 4)

In order to investigate the effect of the low molecular peptide to the fat digestion and absorption, the ratios of lipid digested to the given were measured for 11-day control period on three groups of pigs with each group consisting of three pigs. Then, the testing group I was fed for 12 days with a feed mixture containing a digestive enzyme, the testing group II was fed also for 12 days with a further feed mixture containing the digestive enzyme and also 0.1 wt. % of the low molecular peptide described in the foregoing Experiment 2. The lipid digestion was examined through measurement of amount of lipid contained in the fecus of the animals. The results, as illustrated in Table IV, show that lipid digestion has increased in the control period by 3 and 4 %, respectively, in the reference group and in the group I without the administration of the digestive enzyme and the low molecular peptide, whereas the low molecular peptide has decreased by 8 % in the group II. This means that the addition of the low molecular peptide has resulted in no less than 10 % reduction in the lipid digestion.

(Experiment 5)

As is well known, an excessive fat intake leads to obesity. Then, in this experiment, a high-fat feed containing 30 wt. % of fat added with 1 wt. % of the low molecular peptide of the invention was fed to testing mice for 4 weeks, and body weights and fat tissue weights of the animals were measured. The results, as shown in Table V, indicate apparent reduction in the fat tissue weights in those animals administered with the low molecular peptide in comparison with those without the same. From this fact, it may be readily understood that the lipid metabolism promoting agent of the invention, when added to such high-fat food or feed, may suppress digestion and absorption of fat and may consequently promote lipid metabolism thereby reducing the body fat. A further observation reveals that the animal protein based low molecular peptide is superior in its effect to the plant protein based low molecular peptide.

(Experiment 6)

Chocolate is a highly fatty sweet containing more than 35 wt. % of fat. However, since the fat content significantly accounts for its taste, it has been very difficult to reduce the fat content without sacrificing good taste. Then, the present inventor prepared a special-made chocolate by adding 10 wt. % relative to the fat content of the red cell protein based low molecular peptide of the invention to a commercially available reference chocolate. This specially prepared chocolate was forcibly fed to groups of Beagle dogs. And, measurements were conducted by cross-over method to observe time course of changes in the blood cholesterol and triglyceride values in the animal. Table VI shows the results. Area-under-curve values (AUC, mg hr/dl) and maximum blood concentration values (C max, mg/dl) were obtained in reference to the changes in the blood concentration in the measurement values. As shown, there may be recognized a significant reduction in these values with the group fed with the special chocolate added with the low molecular peptide in comparison with the group fed with the reference chocolate.

(Experiment 7)

In a young yellow tail raising marine farm, a feed added with 0.2 wt. % of the red cell protein based low molecular peptide was fed to the fish for 1 month. Then, through an arbitrary choice, some fish were processed into "sashimi" (sliced raw fish meat). Then, a blind test was conducted among 18 volunteers on the meat taste of those fish fed with the peptide-containing feed and on those fed with the reference feed alone. In the results tabulated in Table VII, tasting impressions of the volunteers were numerically ranked with higher the numerical values the better the tasting impression. As shown, the volunteer testants clearly preferred those fish fed with the peptide-containing feed. More particularly, the volunteers described the meat fed with the reference feed alone with such negative expressions as oily, sticky, smelly or too sour while describing the meat fed with the invention agent as such positive expressions as plain, lean, tasty or the like. That is, the volunteers evaluated highly the fish meat cultured with the administration of the low molecular peptide of the present invention thereby suggesting the reduction in the fat amount and improvement in the fat composition quality due to the invention agent. Further, from this fact, it may be reasonably assumed that administration of the low molecular peptide of the invention will also prevent decomposition of fish meat attributable to excessive body fat content thereby effectively preserving and prolonging the freshness of the product.

6

As may be readily seen from the results of the above-described experiments, through administration of the low molecular peptide with the average amino acid chain length of about 3 to 4 by the dosage amount of 0.5 to 50 % relative to the fat intake, the digestion and absorption of the lipid may be suppressed and further the lipid metabolism may be improved.

The lipid metabolism promoting agent of the present invention may be readily contained as an additive by itself or as blended or mixed with other substances in medicines, food stuff or feed (including both blended feeds and mixed feeds). Especially, when the agent per se is used as food stuff or feed, the agent may be effectively utilized as a physiologically functional food or feed capable of suppressing digestion and absorption of fat and of improving the lipid metabolism. For example, for prevention or treatment of high blood pressure, arteriosclerosis or obesity in the human body, the agent may be used selectively in the form any of medicine, food stuff or a food additive. Similarly, in the case of its application in the fields of livestock or marine products industry, the agent may be used selectively in the form of medicine, feed, or a feed additive.

Especially, for the purpose of prevention or treatment of high blood pressure, arteriosclerosis or obesity in the human body, it is preferred that the

Table IV

| (Experiment 4) | | | |
|---|---|---|---|
| | reference group | group I | group II |
| control period | 68 | 62 | 65 |
| treatment period | 71 | 66 | 57 |
| difference | +3 | +4 | -8 |

Table V

| (Experiment 5) | | | | | | |
|---|---|---|---|---|---|---|
| feed | ♂ | | | ♀ | | |
| | body weight | fat tissue | ratio | body weight | fat tissue | ratio |
| A | 36.5 | 2.0 | 5.5 | 29.0 | 1.8 | 6.0 |
| B | 36.9 | 1.6 | 4.4 | 29.2 | 1.4 | 4.6 |
| C | 34.8 | 1.3 | 3.3 | 29.3 | 1.3 | 4.2 |
| D | 36.9 | 1.8 | 4.8 | 29.3 | 1.4 | 4.9 |
| A....a high fat feed (reference), | | | | | | |
| B....a low molecular peptide obtained from a red cell protein, | | | | | | |
| C....a low molecular peptide obtained from fish powder protein, | | | | | | |
| D....a low molecular peptide obtained from soya protein. | | | | | | |

Table VI

| (Experiment 6) | | | | |
|---|---|---|---|---|
| group | triglyceride | | cholesterol | |
| | AUC | Cmax | AUC | Cmax |
| referece | 445 | 182 | 100 | 27 |
| with red cell peptide | 304 | 126 | 19 | 9 |

Table VII

| (Experiment 7) | | | | |
|---|---|---|---|---|
| sex | number | reference | with red cell peptide | with food additives |
| M | 11 | 16 | 46 | 27 |
| F | 7 | 11 | 13 | 21 |
| total | 18 | 27 | 59 | 48 |

agent be used as a physiologically functional food stuff, since this usage permits ready control of intake proportion relative to a total fat intake. Moreover, in the field of marine product industry where in-body fat growth or accumulation may be easilly assessed through a dissection or observation of sampled fish body, the fat amount in the animal meat may be readily improved by controlling the administration amount of the lipid metabolism promoting agent of the invention in accordance with the assessed growth or accumulation of the body fat in the animal meat.

As described above, the lipid metabolism promoting agent and its usage relating to the present invention is suitable for preventing or treating high blood pressure or arteriosclerosis or for improving meat quality of cattles or fish in the field of the livestock industry and the marine products industry.

**Claims**

1. Use of a mixture of low molecular peptides prepared through hydrolysis of a protein or protein-containing material by a protease or an acid, the mixture having an average peptide chain length of approximately 3 to 4, for producing a lipid metabolism promoting agent being effective for decreasing triglyceride concentration and reducing fat digestion and absorption.

2. Use of a mixture of low molecular peptides as claimed in Claim 1, wherein said protease comprises a combination of any one or more of an acid protease, a neutral protease or an alkaline protease.

3. Use of a mixture of low molecular peptides as claimed in Claim 1, wherein said acid comprises an inorganic acid such as hydrochloric acid or sulfuric acid.

4. Use of a mixture of low molecular peptides as claimed in Claim 1, wherein said low molecular peptide is obtained through hydrolysis of a mixture containing 1 mg of protein relative to 0.1 to 5 units of protease, said hydrolysis being carried out for 3 to 48 hours at the temperature of 20 to 70 degrees Celsius.

**Patentansprüche**

1. Verwendung eines Gemisches aus niedermolekularen Peptiden, die durch Hydrolyse eines Proteins oder eines proteinenthaltenden Materials mit Hilfe einer Protease oder einer Säure hergestellt werden, wobei das Gemisch eine durchschnittliche Peptidkettenlänge von ungefähr 3 bis 4 aufweist, zur Herstellung eines Mittels zur Verbesserung des Fettabbaus, das die Wirkung hat, die Triglyceridkonzentration zu verringern und die Fettverdauung und -aufnahme zu reduzieren.

2. Verwendung eines Gemisches aus niedermolekularen Peptiden nach Anspruch 1, wobei die Protease eine Kombination irgendeiner oder mehrerer Säureproteasen, neutraler Proteasen oder basischer Proteasen beinhaltet.

3. Verwendung eines Gemisches aus niedermolekularen Peptiden nach Anspruch 1, wobei die Säure eine anorganische Säure wie zum Beispiel Chlorwasserstoffsäure oder Schwefelsäure beinhaltet.

4. Verwendung eines Gemisches aus niedermolekularen Peptiden nach Anspruch 1, wobei die niedermolekularen Peptide durch Hydrolyse eines Gemisches erhalten werden, das 1 mg Protein im Verhältnis zu 0,1 bis 5 Einheiten Protease enthält, wobei die Hydrolyse mit einer Dauer zwischen 3 und 48 Stunden bei einer Temperatur zwischen 20 und 70 Grad Celsius ausgeführt wird.

**Revendications**

1. Utilisation d'un mélange de peptides de faible masse moléculaire préparé par hydrolyse d'une protéine ou d'un produit contenant des protéines par une protéase ou un acide, le mélange ayant une longueur moyenne de chaîne peptidique d'approximativement 3 à 4 acides aminés, pour la production d'un promoteur du métabolisme des lipides qui est efficace pour faire diminuer la concentration en triglycérides et réduire la digestion et l'absorption des graisses.

2. Utilisation d'un mélange de peptides de faible masse moléculaire selon la Revendication 1, dans lequel ladite protéase comprend une combinaison d'une ou plusieurs quelconques protéases acides, protéases neutres ou protéases alcalines.

3. Utilisation d'un mélange de peptides de faible masse moléculaire selon la Revendication 1 dans lequel ledit acide comprend un acide minéral tel que l'acide chlorhydrique ou l'acide sulfurique.

4. Utilisation d'un mélange de peptides de faible masse moléculaire selon la Revendication 1 dans lequel ledit peptide de masse moléculaire est obtenu par hydrolyse d'un mélange contenant 1 mg de protéines pour 0,1 à 5 unités de protéase, ladite hydrolyse étant effectuée pendant 3 à 48 heures à une température de 20 à 70°C.